# EUROPEAN PATENT APPLICATION

(11) **EP 1 077 054 A2**
(43) Date of publication of application: **21.02.2001**
(21) Application number: 00117745.0
(22) Date of filing: 17.08.2000
(51) Int. Cl.: A61F 13/49, A61F 13/64

(54) **Absorbent article**

(30) Priority: 18.08.1999 US 376282
(71) Applicant: First Quality Enterprises, Inc., McElhattan, Pennsylvania 17748 (US)
(72) Inventor: Damaghi, Kambiz, Kings Point, New York 11021 (US); Turi, Mordechai, Conyngham, PA 18219 (US); Kauschke, Michael, Yonkers, New York 10701 (US); Karami, Hamzeh, McElhattan, PA 17748 (US)
(74) Representative: Motsch, Andreas

(57) **Abstract**

An absorbent garment comprises a front waist region, a back waist region, a crotch region, a pair of spaced apart leg openings and a fastening region intermediate the crotch region and the front region. Elasticized band members having end portions extend angularly from the back waist region toward the fastening region, with said end portions engaging one another and onto the fastening region for more perfect fit around the torso of the wearer. In different embodiments, the absorbent garment is generally T-shaped, or L-shaped in configuration having male-female fasteners (or a female surface) which, during use, are adapted to inter-engage in one another in order to enhance body fitness and insure against fluid leakage.

## Description

### RELATED APPLICATIONS

This application is a continuation-in-part of application serial number 09/149,265 filed September 8, 1998, which is, in turn a continuation-in-part of application serial number 09/097,198 filed June 12, 1998.

### FIELD OF INVENTION

The present invention relates generally to absorbent articles such as disposable diapers, infant training pants, adult incontinent underpants and briefs used for absorption and containment of urine and other body exudates. More particularly, the present invention is directed to improving body fit and leakage prevention of these garments when worn by infants as well as adults. In one particular aspect, this invention relates to garments of the types herein described which are generally T-shaped in configuration when viewed in stretched position, and which are provided with fastening means uniquely positioned to achieve improved fit around the torso of the wearer, and which also affords optimum leakage prevention.

### BACKGROUND OF THE INVENTION

The aforementioned application serial number 09/149,265 filed September 8, 1998 discloses a diaper structure which has triple member closure designed to insure proper distribution of the pulling forces thus resulting in a more perfect and snug fit to the body of the wearer. The absorbent article described in said application serial number 09/149,265 comprises an absorbent body having a front waist portion, a back waist portion, a crotch portion, a pair of spaced apart leg openings, and a fastening region intermediate the crotch portion and the front waist portion. An elasticized band member is attached to the back waist portion and has a left hand band portion and a right hand band portion, both extending angularly from the area of the back waist portion toward the fastening region. The ends of each of the band portions are adapted to be engaged to one another and to the fastening region, and they may be tensioned to securely fasten the absorbent article to the body of the wearer.

While the diaper structure described in said application constitutes an advancement over the prior art diapers and briefs, there is still room for further improvements in the design and/or configuration of the diaper.

Accordingly, it is an object of this invention to provide an improved disposable absorbent garment such as diapers, briefs, adult incontinent underpants, guards and the like, hereinafter also referred to as "garments" and described specifically by reference to diapers.

It is also an object of this invention to provide such absorbent garments which lend themselves to simplicity of fabrication and assembly and which are remarkably effective against leakage of fluid and other body exudates.

It is a further object of this invention to provide an absorbent garment of the type, and for the purpose hereinafter described which has a generally T-shaped configuration when the garment is viewed in stretched position, and which is provided with strategically positioned male-female engagement means so as to provide body-tight but comfortable fit around the torso of the wearer.

The foregoing and other features of the present invention will be more fully appreciated from the ensuing detailed description and the various figures in the accompanying drawings all of which form parts of this application.

### SUMMARY OF THE INVENTION

In order to improve on the containment characteristics and body fitness of absorbent garments, the present invention provides absorbent articles having a chassis with a front waist region, a back waist region, a crotch region, a pair of leg openings and a fastening region intermediate the crotch region and the waist region. A pair of angularly disposed band portions extend from the left and right of the back waist region, each band portion having an end portion which overlap each other and engage onto the fastening region which acts like a landing zone.

In another embodiment of the invention, the absorbent article has a generally T-shaped configuration and comprises a first portion and a second portion disposed vertically with respect to the first portion. The first portion has opposed lateral ears or flaps, with one flap having a male fastener and the other flap having a female fastener adapted to engage into one another when the two portions are wrapped around the torso of the wearer and onto each other. The second portion has a proximal end and a distal end, and a pair of spaced apart male fasteners on the surface of the distal end. The male-female fasteners are spaced apart and aligned such that when the second portion is passed under the crotch and folded upwardly thereover and onto the overlapped segments of the first portion, each male fastener on the second portion engages a correspondingly aligned female fastener on the surface of the first portion. If desired, the first portion may be made of a nonwoven fabric having a female surface adapted for engagement with the male fasteners, in which case the female fasteners are not required.

In still another embodiment, the absorbent garment has a generally L-shaped configuration with male-female fasteners, or with male fasteners and a female surface, as hereinafter described in more detail.

### BRIEF DESCRIPTION OF THE DRAWINGS

In the drawings, wherein like reference numerals are employed to represent like parts wherever possible:
Figure 1 is a perspective view of a pant-type absorbent article made in accordance with one embodiment of the present invention having elastic band (belt) and a triple member closure in the front waist region, in fastened position;
Figure 1 A is similar to Figure 1 except that two separate elastic band members are used to form the triple member closure;
Figure 2 is a plan view of the front of the absorbent article shown in Figure 1;
Figure 3 is a stretched plan view of the absorbent article in Figure 1 showing the elastic band angularly disposed at both ends, from the hip toward the front crotch portion;
Figure 3A is a sectional view taken along the line 3A-3A of Figure 3;
Figure 4 is a plan view of the left side of the absorbent article shown in Figure 1;
Figure 5 is a stretched plan view of the absorbent article similar to Figure 3 but wherein the ends of the elastic band are angularly disposed from the center of the back waist portion toward the front of the crotch portion;
Figure 6 is a plan view of the left side of the absorbent article shown in Figure 1;
Figure 7 is another stretched plan view similar to Figure 3 but wherein the ends of the elastic band are angularly disposed curvilinearly from the hip toward the front crotch portion;
Figure 8 is a plan view of the left side of the absorbent article in Figure 1 with the ends of the elastic band disposed curvilinearly as in Figure 7;
Figure 9 is a stretched plan view similar to Figure 7 but wherein the ends of the elastic band are angularly disposed curvilinearly from the center of the back waist portion toward the front of the crotch portion;
Figure 10 is a plan view of the left side of the absorbent article shown in Figure 1 with the ends of the elastic bands disposed curvilinearly as shown in Figure 9;
Figure 11 is stretched plan view of an absorbent article similar to Figure 9 but wherein the elastic elements are formed into respective loops attached to the elastic band;
Figure 12 is a cross sectional view taken along the line 12-12 of Figure 2, in fastened position, illustrating the different layers of the absorbent pad and the triple member closure region;
Figure 13 is an enlarged view of portion of the triple closure member region, in fastened position;
Figure 14 is an enlarged view similar to Figure 13 but wherein the triple member closure is shown in unfastened position;
Figure 15 is a perspective view of a different embodiment of an absorbent article having a triple member closure similar to the absorbent article shown in Figure 1 but wherein a separate elastic band is attached to the center of the back waist portion of the article;
Figure 16 is a plan view of the front of the absorbent article shown in Figure 15 with the separate elastic bands in fastened position;
Figure 17 is a perspective view of the absorbent article shown in Figure 15 but wherein the separately attached elastic band is in open (unfastened) position ;
Figure 18 is a plan view of the front of the absorbent article shown in Figure 17;
Figure 19 is a cross sectional view taken along the line 19-19 of Figure 18;
Figure 20 is a stretched plan view of the absorbent article shown in Figure 17;
Figure 21 is a perspective view of an absorbent article made according to another embodiment of this invention having triple member closure fastened in the front portion, and separate elastic band attached to the rear portion of the absorbent pad of the article;
Figure 22 is a stretched plan view of the absorbent article shown in Figure 21 but wherein the separate elastic band is in open (unfastened) position;
Figure 23 is a perspective view of the embodiment of the invention directed to an absorbent garment having a generally T-shaped configuration in assembled form;
Figure 24 is a perspective view of the embodiment which is similar to Figure 23 showing the vertical center portion of the garment partially folded up and out;
Figure 25 is a perspective side view of the absorbent garment illustrated in Figure 24 showing the vertical center portion of the garment hanging freely prior to assembly of the article;
Figure 26 is a stretched view of the garment shown in Figure 23;
Figure 27 is a sectional view taken along the line 27-27 of Figure 26;
Figure 28 is a stretched view of the garment similar to Figure 26 but illustrating a different embodiment of the fastening means; and
Figure 29 is a stretched view of yet another embodiment of the invention.

### DETAILED DESCRIPTION OF THE DIFFERENT EMBODIMENTS OF THE INVENTION

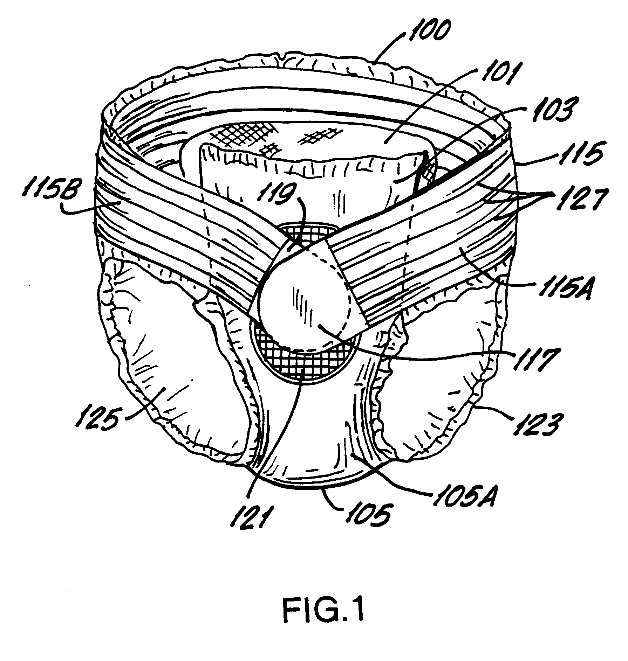

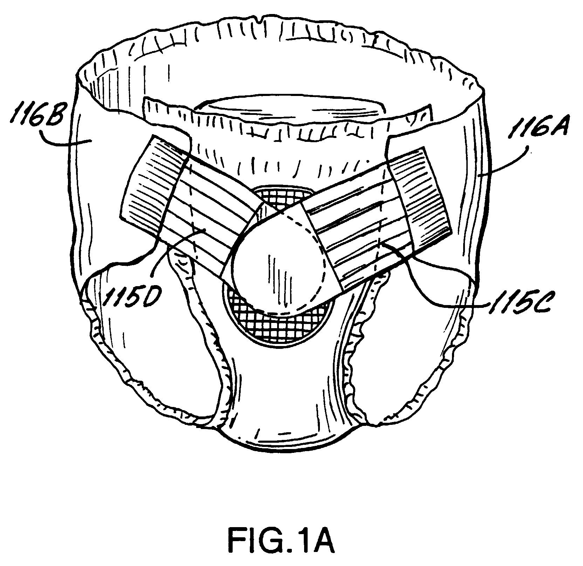

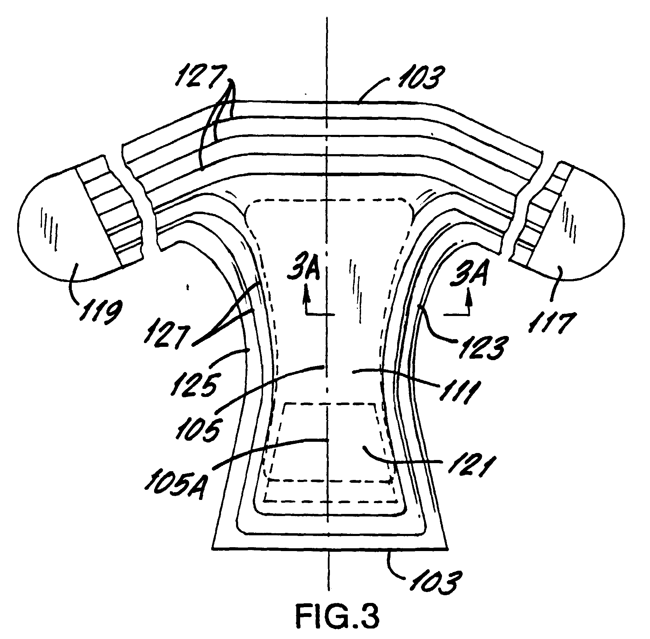

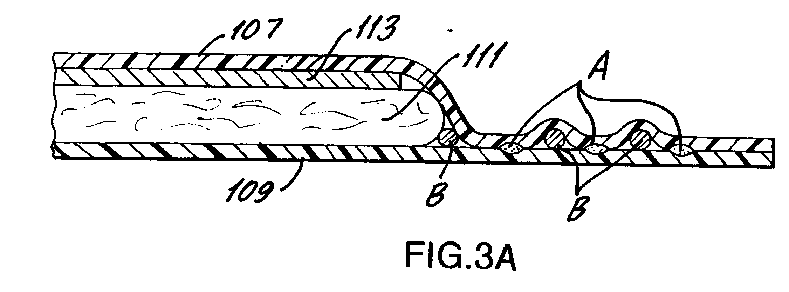

Referring first to Figure 1 of the drawings, there is shown an absorbent article in the form of a brief, generally designated as 100. The term "brief" as used herein is intended to refer to disposable garments worn below the lower part of the torso by incontinent persons and also comprises disposable articles such as baby diapers, adult incontinent underpants, guards and the like articles. The absorbent article 100 comprises a back waist region 101, a front waist region 103 and a crotch region 105 having a front crotch portion 105A. Referring to Figure 3A, the absorbent article 100 comprises a liquid pervious top sheet or layer 107 facing the body of the wearer, a liquid impervious backsheet 109 which is usually coextensive with the top layer 107, and an absorbent layer or pad 111 disposed between the top sheet 107 and backsheet 109. An acquisition layer 113 is disposed between the top sheet 107 and the absorbent layer 111 and serves to temporarily retain the body exudates and slowly distribute them through the absorbent pad 111. These layers are sealed at their corresponding ends to form a composite sheet.

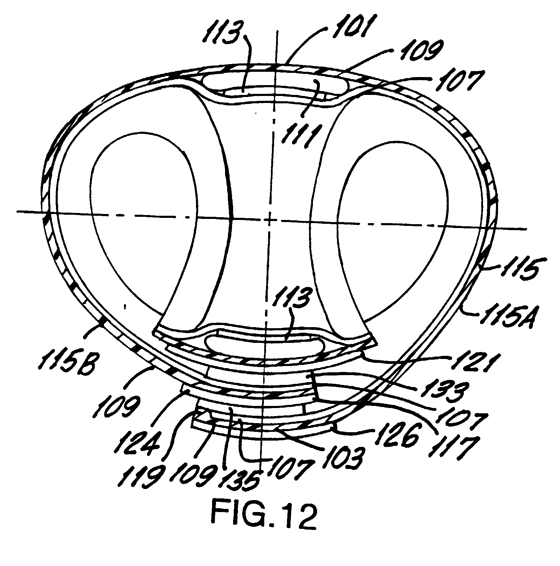

The absorbent article 100 shown in Figure 1 has an elastic band or belt 115 which is attached to the back waist portion 101 and extends around the waist angularly toward the crotch region as a left hand belt portion 115A and right hand belt portion 115B. As shown in Figures 12, 13 and 14 the left hand belt portion 115A terminates in a fastenable closure end 117 and the right hand belt portion 115B terminates in the fastenable closure end 119. These ends are adapted to be engaged to one another and to the fastening region 121 in the front of the article thereby providing a triple member closure defined by inter-engagement of the respective ends 117, 119 of the belt portions 115A-115B, and the fastening region 121. Thus, each end portion (117, 119) has an inner adherent surface 133, 135 and an outer non-adherent surface 124, 126. The fastening region 121 is also a non-adherent surface, therefore, in fastened position the inner adhering surface of one end is securely adhered to the outer non-adhering surface of the other end, and the inner adhering surface of the other end is securely adhered to the non-adhering surface of the fastening region to form a triple member closure as shown in Figures 12, 13 and 14. There is also shown in Figure 12 (fastened position) the liquid pervious backsheet 107 and the liquid impervious layer 109 and the absorbent layer 111 interposed therebetween.

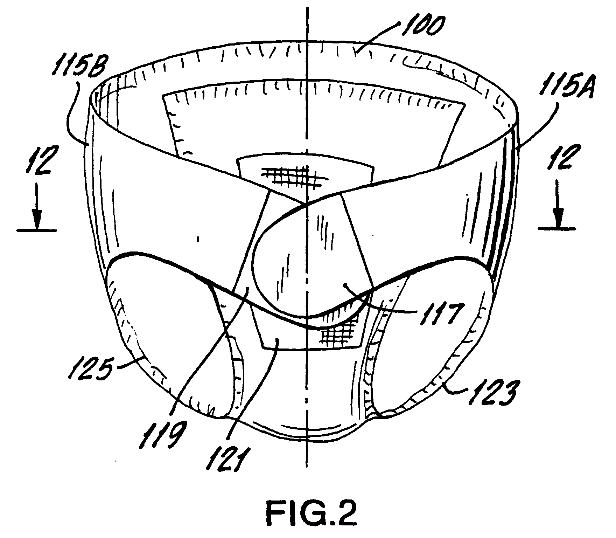

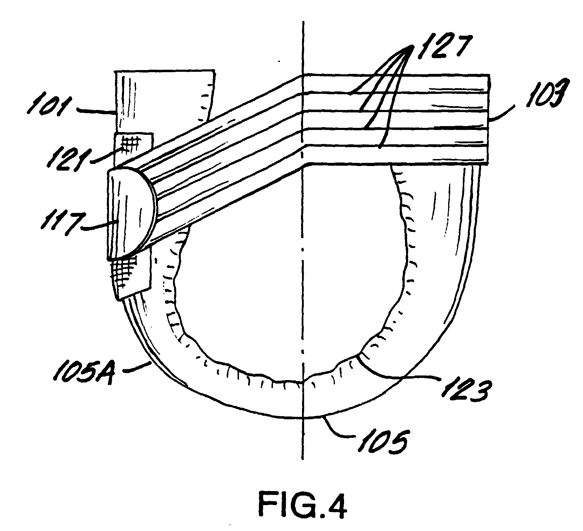

The absorbent article 100 also comprises the leg openings 123, 125, and the belt portions 115A, 115B each comprises elastic elements 127 which impart elasticity to the article. Alternatively, the elastic belt 115 itself may be made of an elastic material in which case the elastic elements 127 are not required. Both belt portions 115A, 115B of the elastic belt 115 are disposed angularly toward the crotch region 105 as is further illustrated in Figures 2, 3, 4-10. Figure 4 shows the angular disposition of the elastic band or belt relative to the horizontal axis of the article. This angle, may vary depending on the body shape and the location of the fastening region 121, which itself may constitute a large or small area as desired.

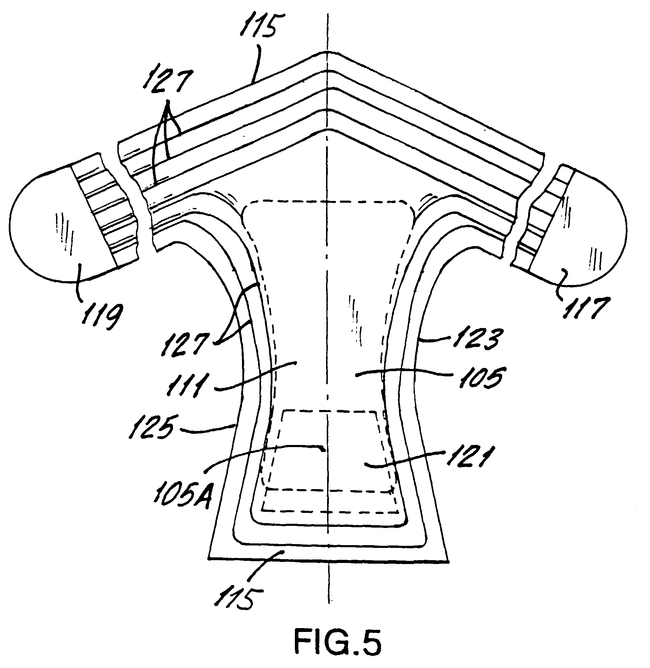

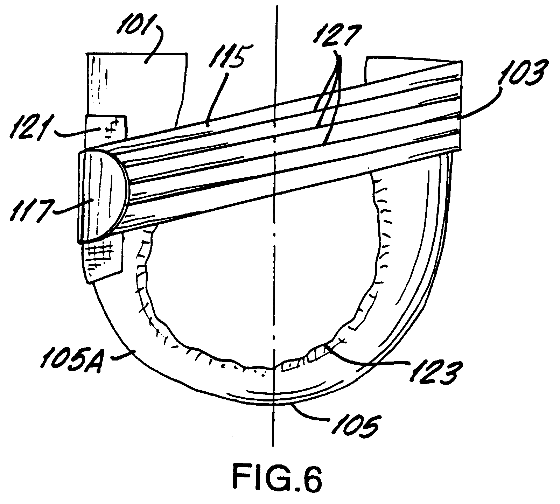

In Figure 5, the elastic belt 115, and hence the elastic elements 127 of the belt, are angularly disposed from the center of the back waist portion of the article toward the front crotch region. This construction is further illustrated in Figure 6.

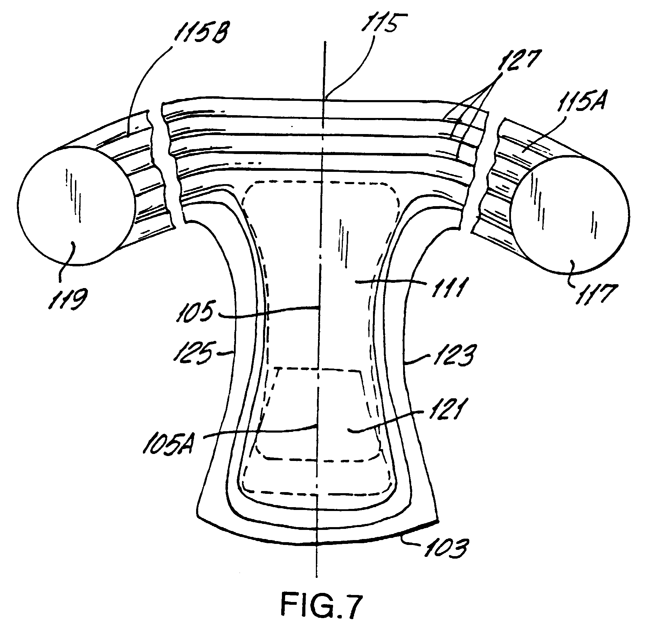

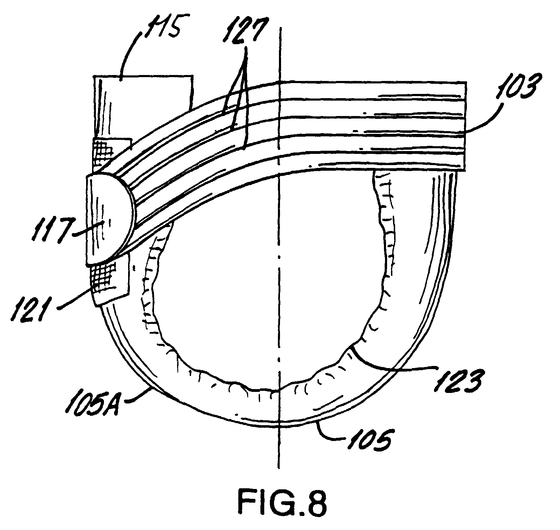

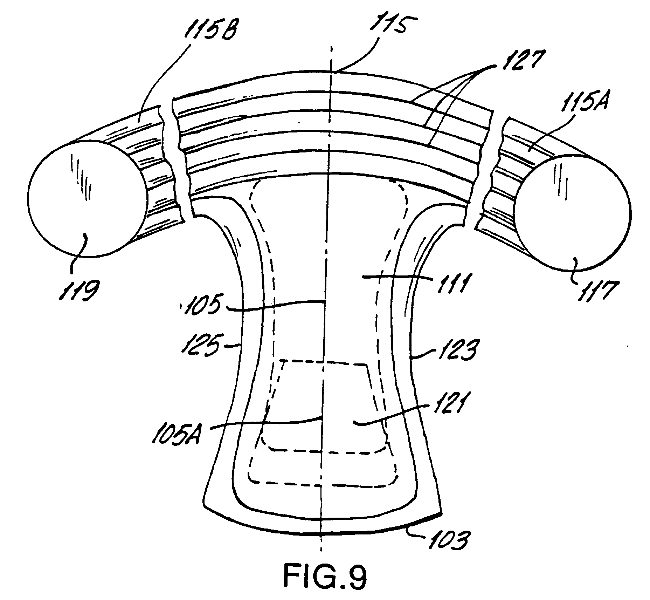

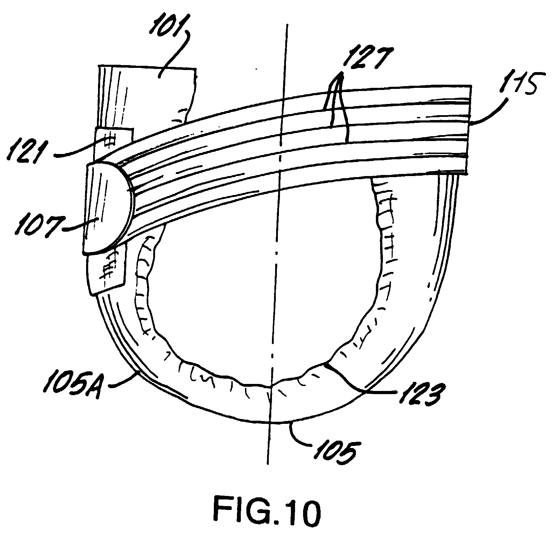

In Figures 1-6, the elastic elements 127 of the elastic belt 115, and its respective band portions 115A, 115B are shown extending linearly, i.e., in relatively straight substantially parallel lines angularly toward the crotch region. In Figures 7-10, the elastic elements are shown to extend curvilinearly, at an angle relative to the horizontal axis of the article. Thus, in Figures 7 and 8 the elastic elements 127 extend curvilinearly, at an angle, from the hip toward the front of the crotch region, and in Figures 9 and 10 the elastic elements 127 are disposed angularly curvilinearly from the center of the back waist portion toward the front of the crotch region.

While reference is made to linear or curvilinear elastic belt, it should be mentioned that these refer to the fabricated, pre-use appearance of the elastic belt since in use, and as a practical matter, the elastic belt conforms to the contours of the body of the wearer.

Also, while the elastic belt portions 115A, 115B are shown extending angularly from the hip to the front crotch region (see Figures 3, 4, 7 and 8), these elastic belt portions may, in a different variation, extend from the center of the back waist portions to the front crotch region (see Figures 5, 6, 9 and 10), or the elastic belt may extend from any point between the center of the back waist and the hip toward the crotch region. All these structures and configurations are within the scope of the present invention.

As shown in Figure 1 A, the elastic band need not be a continuous band but rather, one could use two separate elastic band members 115C, 115D which may be secured to the left and right hips at 116A, 116B respectively. Otherwise, the structure of the absorbent article is the same as in Figure 1.

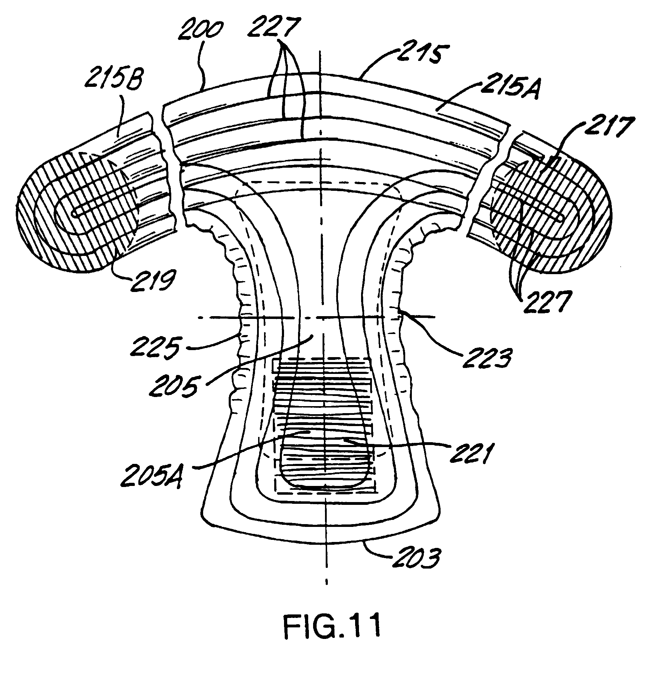

Referring to Figure 11, the absorbent article 200 shown therein is similar in structure to the absorbent article 100 shown in Figure 3 except that the elastic elements are formed into respective loops 227 A. Also as in the absorbent article shown in Figures 1-3, the absorbent article 200 has an elastic band or belt 215 which extends around the waist as in the previous embodiment, i.e., into a left hand belt portion 215A and a right hand belt portion 215B. However, the elastic elements 227 of the elastic belt 215 are formed into loops which span from the end 217 to the end 219 of the respective belt portions 215A and 215B. At its front, the absorbent article 200 has a fastening region 221, and the leg openings 223 and 225. As in the previous embodiments, each of the ends of the belt portions 215A and 215B has an outer non-adherent surface and an inner adherent surface for fastening and unfastening of the ends of the belts relative to each other and to the fastening region 221 which has an outer non-adherent surface.

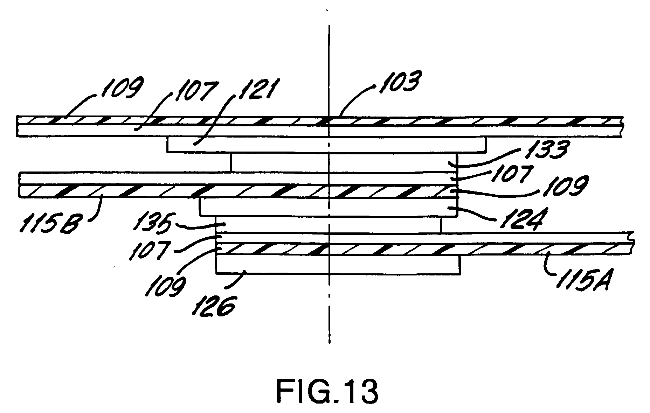

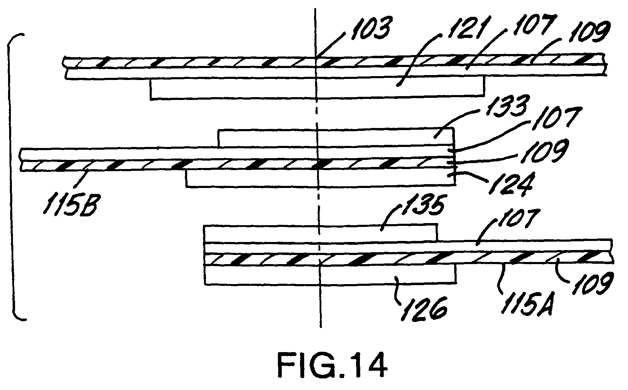

It must be noted that the layered structure depicted in Figures 13 and 14 is the same for all embodiments of the invention and are numbered uniformly for the sake of consistency.

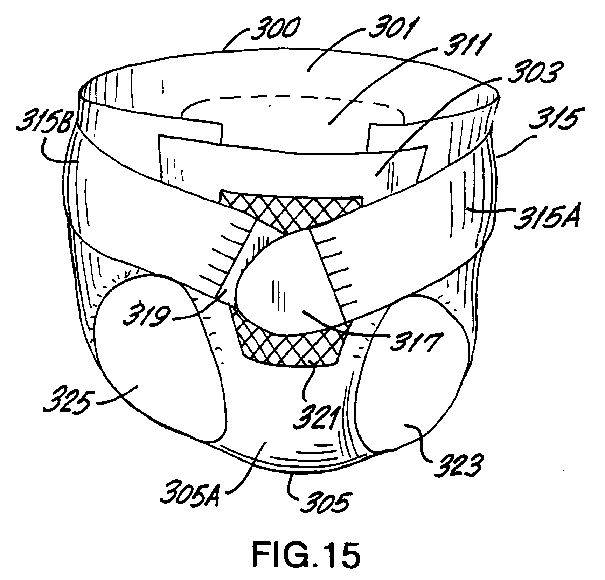

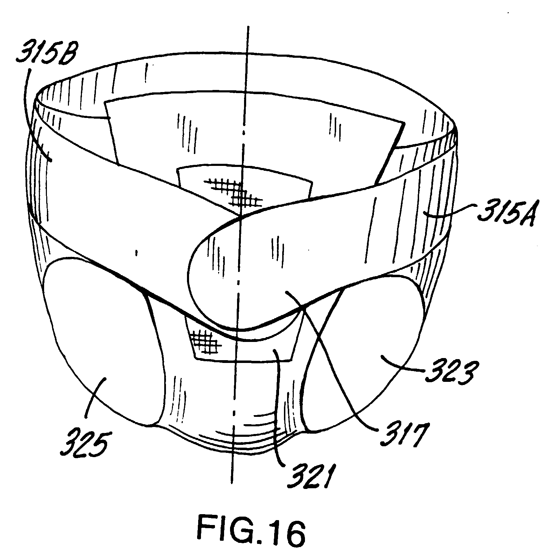

In Figures 15 and 16, the absorbent article 300 has a back waist portion 301 and a front waist portion 303, and is provided with a separate elastic band 315 attached to the backwaist portion 301. As in the previously described embodiments the elastic belt 315 comprises the left hand elastic belt portion 315A and the right hand elastic belt portion 315B having their respective ends 317 and 319 attached to the fastening region 321. As in the previous embodiments the absorbent article 300 comprises the absorbent panel 311, the leg openings 323 and 325 and the crotch region 305 having a front crotch region 305A.

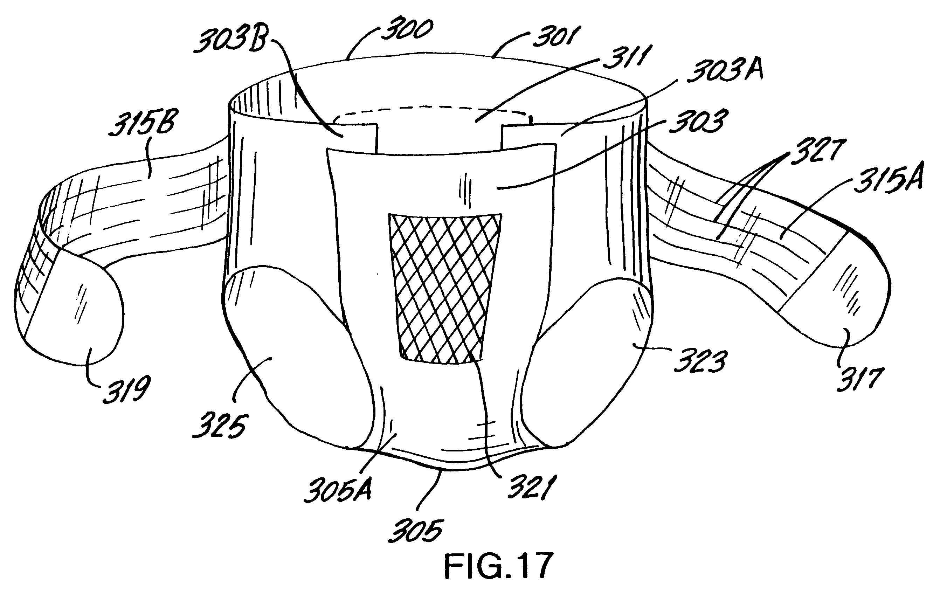

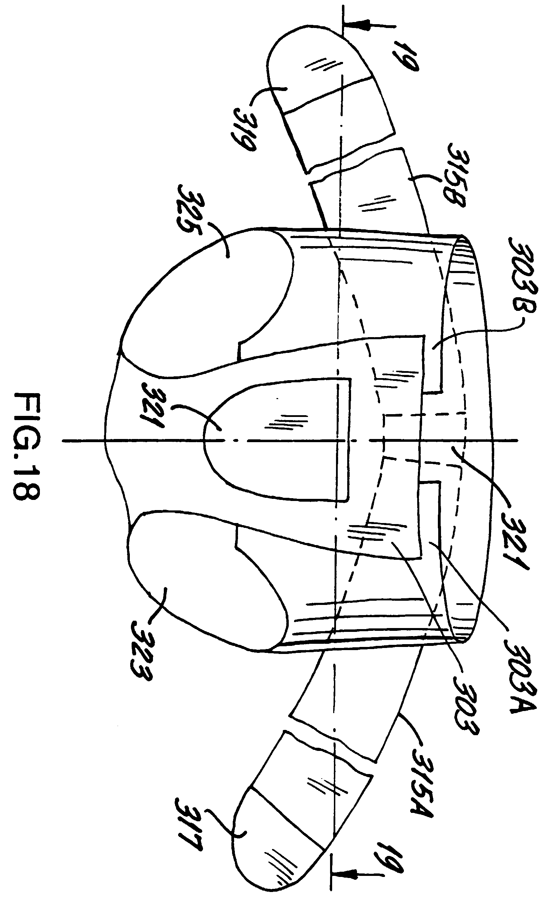

Figures 17 and 18, similar to Figures 15 and 16, respectively, except that the ends of the elastic belt are in unfastened position. Thus, as shown in Figure 17, the absorbent article 300 comprises a back waist portion 301, a front waist portion 303 having a left hand front waist portion 303A a right hand front waist 303B, a crotch region 305, a front crotch region 305A, a fastening region 321 and leg openings 323, 325.

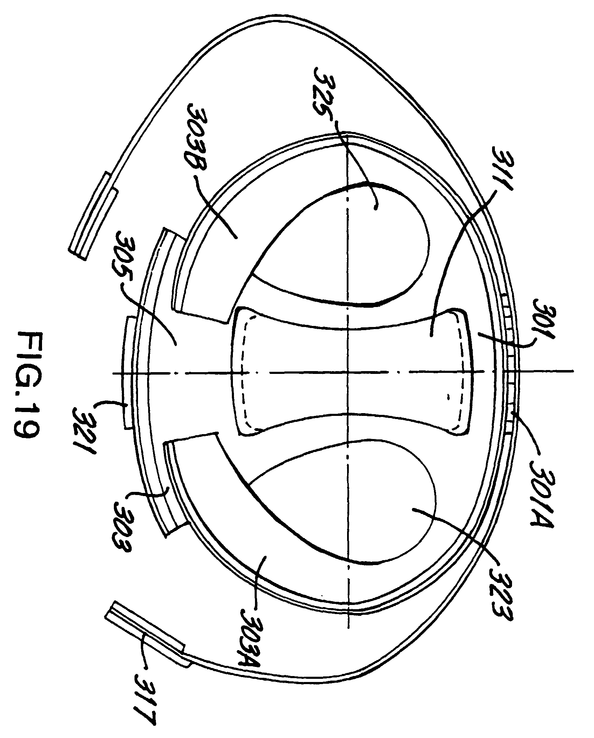

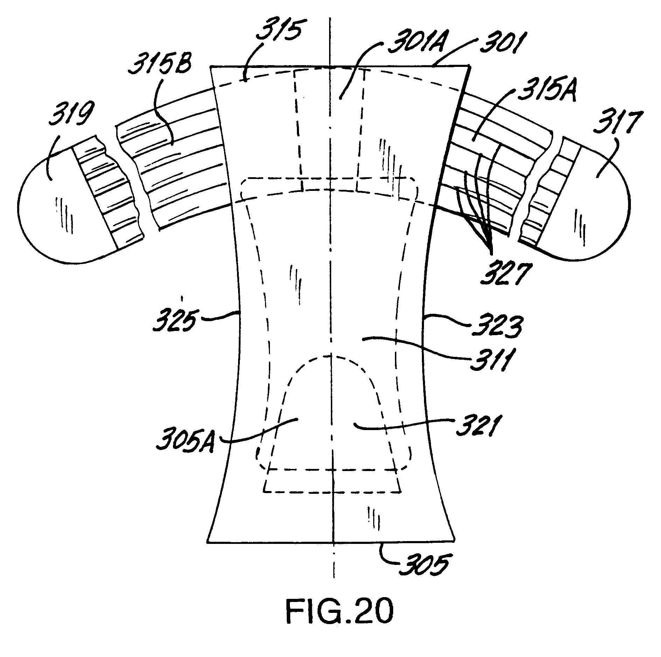

Figures 19 and 20 show more clearly how the separate elastic belt 315 is attached to the back waist attachment region 301A of the backwaist portion 301 of the absorbent article and extends as the left and right elastic belts portions 315A, 315B angularly disposed toward the fastening region 321. As in the previous embodiments, the ends 317 319 of the elastic belt portions 315A, 315B are adapted to adherently overlap on one another and on the fastening region 321 to form a triple member closure. In the embodiment shown in Figure 19, the left hand belt portion 317 overlaps the right hand portion 319, however the overlapping positions of the ends 317,319 may be reversed. Alternatively, the ends of the belts may be simply tied together if desired.

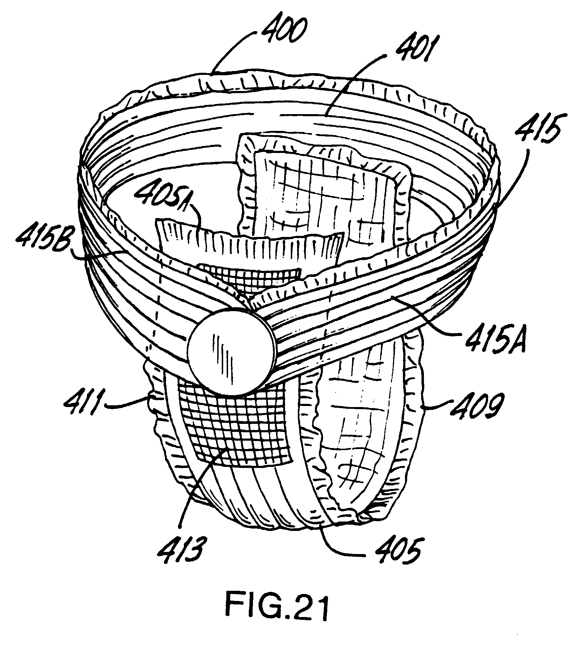

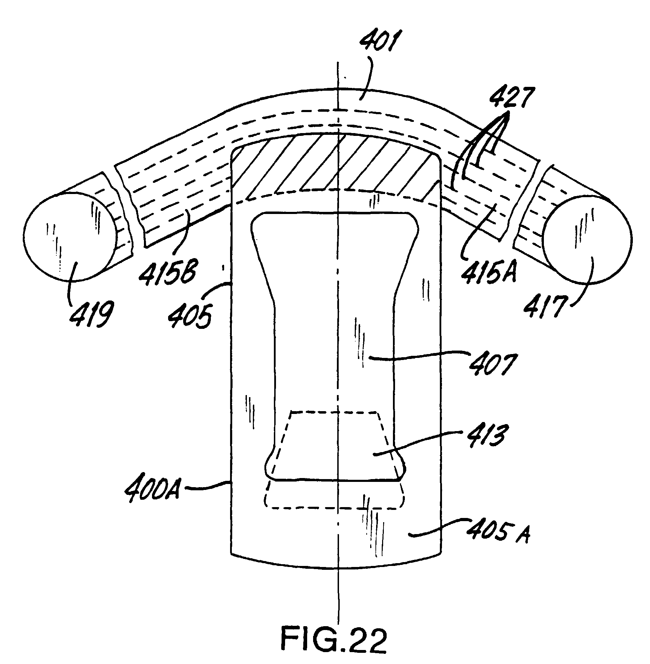

In Figures 21 and 22, the absorbent article 400 is a two piece construction basically consisting of a separate elastic belt and absorbent body portion. The body portion 400A comprises a back waist portion 401, a front waist portion 403, a crotch region 405, a front crotch region 405A, an absorbent layer or pad 407, the leg openings 409 and 411 and a front waist fastening region 413. The absorbent article shown in Figures 21 and 22 also has a separate elastic band or belt 415 which is attachable at the back waist portion 401 around the waist portion and extends angularly toward the crotch region as a left hand belt portion 415A and a right hand belt portion 415B. The left hand belt portion 415A terminates in the end 417 and the right hand belt portion 415B terminates in the end 419. As in the previously described embodiments, the ends 417 and 419 are adapted to engage to one another and to the front waist fastening region 413 to form a triple member closure.

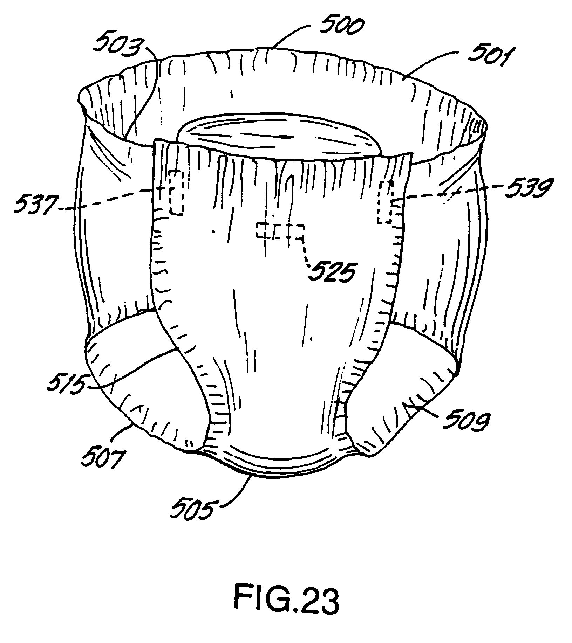

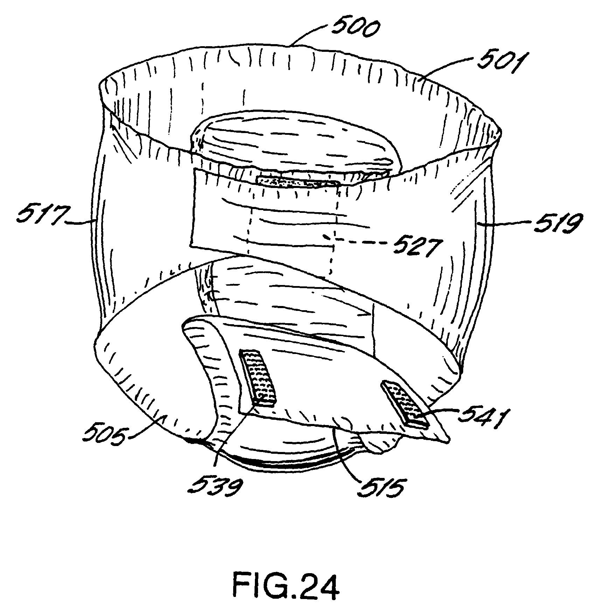

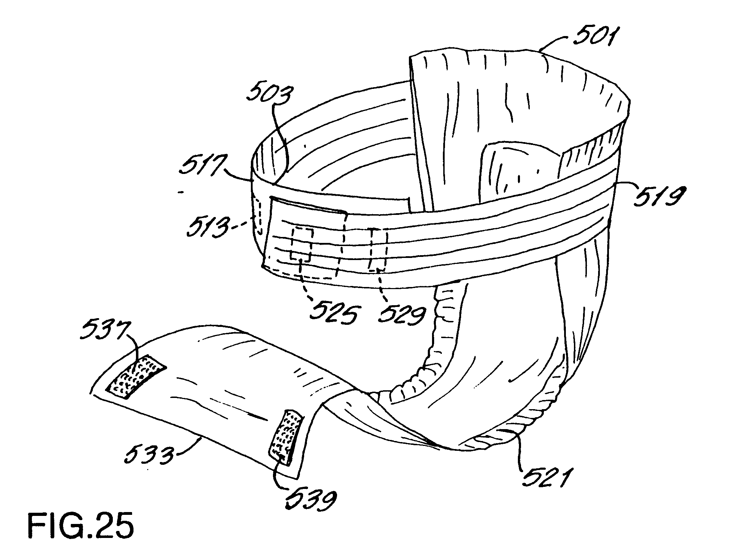

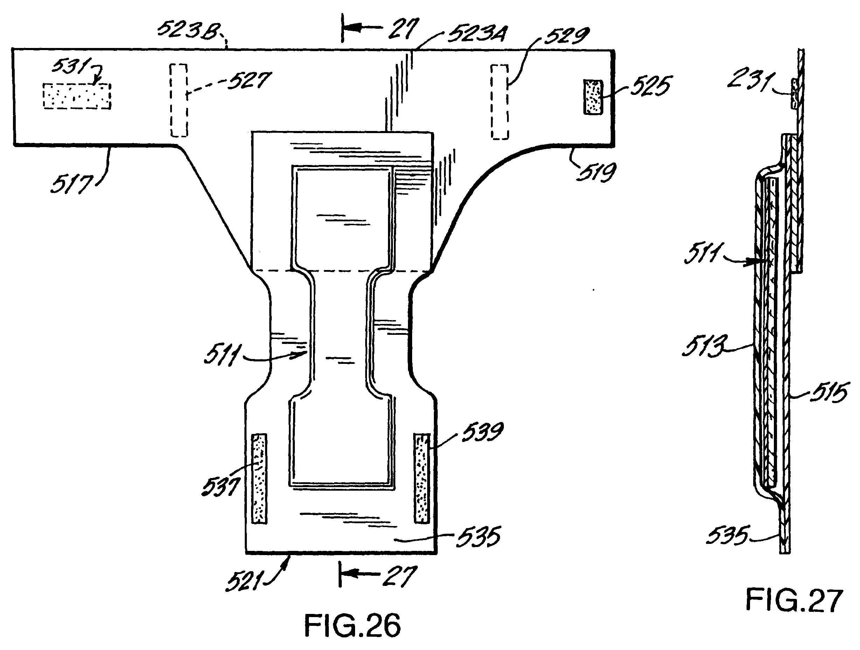

Referring now to the embodiment of the invention which is directed to the generally T-shaped variant of the absorbent garment (diaper) shown in Figures 23-27, there is shown, in Figure 23, a diaper generally designated as 500 comprising a chassis having a back waist region 501 and a front waist region 503 ( which may be elasticized), a crotch region 505 and a pair of leg openings 507, 509 through which extend the legs of the wearer. The diaper 500 also comprises an absorbent core or pad 511 which is disposed between a liquid pervious cover sheet 513 and a liquid impervious backsheet 515 as illustrated in Figure 27 and described hereinafter. The back waist region 501 comprises a mid region 523 having a pair of laterally extending segments or ears 517, 519 and a center segment or flap 521 which extends vertically downward relative to the ears 517, 519 as illustrated in Figures 25 and 26. The ear segments 517, 519 are adapted to be wrapped around the waist, and the center segment 521 is adapted to be passed under the crotch region 505, pulled up and folded over the crotch region 505 and engaged onto the ear segments 517, 519 as hereinafter described. In order to assure a more perfect fit of the diaper around the torso of the wearer, the ear segments 517, 519 and the center flap 521 are provided with fastening means at strategic locations on their respective surfaces. For example, in the embodiment shown in Figure 26, the front surface 523A of the mid region 523 is provided wit a male Velcro-type fastening means 525 adjacent the right lateral end of the ear segments 519, and the reverse surface 523B opposite the surface 523A is provided with a female Velcro-type fastening means 527, 529, 531. The distal end 533 of the center segment 521 has a top surface 535 on which is provided a pair of opposed, generally parallel and spaced apart male Velcro-type fastening means 537, 539. As it will become apparent from the description of the assembly of the diaper during its use, the fastening means on the ear segments and the center segment are located at such positions as the result in a remarkable tight fit diaper which is highly effective against leakage of fluid and body exudates.

In use, the ear segment 517 is wrapped around the waist first, followed by wrapping the ear segment 519 around the waist to overlap the ear segment 517, and the male Velcro fastener 525 is engaged onto the female Velcro fastener 531. The center segment 521 is then passed under the crotch of the wearer and folded thereover and upwardly onto the top surface 523A of the diaper, and then engaging the male Velcro fasteners 537 and 539 onto the corresponding aligned female Velcro fasteners 527 and 529, respectively. By proper spacings and alignments of the male/female Velcro fasteners, the fasteners will mate, i.e., inter-engage, thus resulting in a more perfect fit to the waist of the wearer and prevent leakage of the body fluids and exudates out of the diaper.

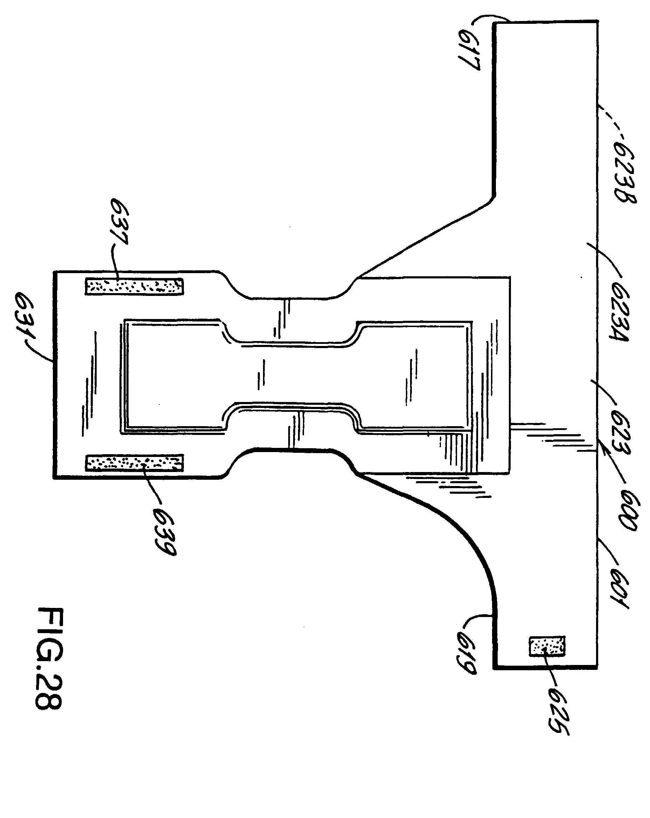

In the embodiment of the invention illustrated in Figure 28 the diaper 600 comprises a back waist region 601 having a mid-region 623 with front and rear surfaces 623A, 623B, and laterally extending ear segments or flaps 617, 619 as in the embodiment shown in Figure 26. However, in the embodiment of Figure 28, the mid region 623 and the ear segments 617, 619 are made partly or entirely from a nonwoven fabric, and thus they can act as female surface for engagement by a male Velcro fastener. In this variant of the invention, the ear segments 617, 619 are wrapped around the waist region as hereinbefore described, and the male Velcro fastener 625 is engaged onto the female fastener-receiving surface, i.e., the surface of the ear segment 617. Thus, no female Velcro fastening means on the surface of the ear segment 617 is necessary. Furthermore, no female Velcro fastening means (such as the female Velcro fastening means 529, 531 shown in Figure 26) are required since the male Velcro fastening means 637, 639 on the center segment 631 can be engaged onto the female receiving surface 623A of the back waist region.

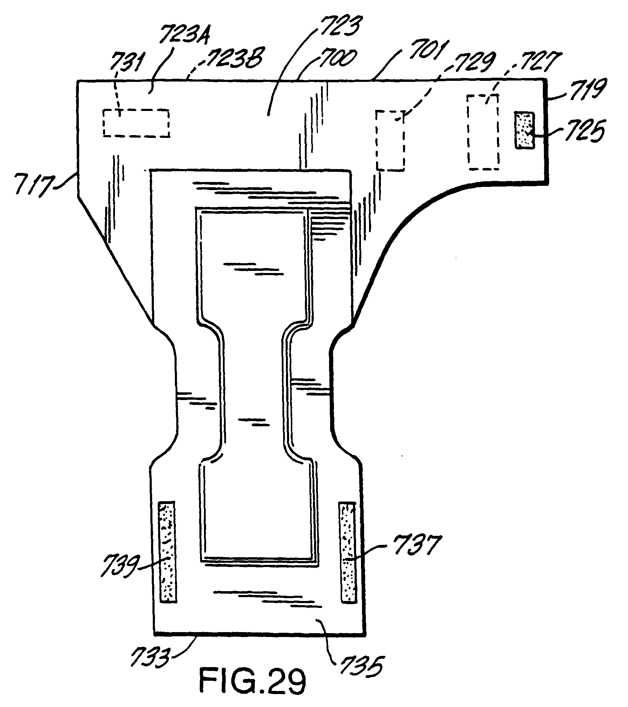

In still another embodiment of the invention shown in Figure 29, the diaper is shown as a somewhat modified T-shaped, generally in an angle iron or L configuration. In this embodiment the diaper 700 comprises a back waist region 701 having a mid region 723 with front and rear surfaces 723A, 723B, a laterally extending ear segment or flap 719, a vertically disposed center portion 733 having a flared out portion 717 as shown in Figure 29. As it can be seen from this figure, the flared out portion 717 does not extend laterally to the same extent as the ear segment 719 and hence the diaper, in stretched view, assumes a modified L-shape structure.

The diaper 700 of this variant of the invention is provided with a male Velcro fastener 725 adjacent the end of the ear segment 719 and female Velcro fasteners 727, 729 and 731. As in the previously described embodiments, the center portion 733 is provided, on its top surface 735, with a pair of opposed and spaced apart male Velcro fasteners 737, 739 at the distal end of the center portion 733.

In use, the ear segment 719 is wrapped around the waist so as to engage the male Velcro fastener 725 onto the female Velcro fastener 731. By proper spacing of the Velcro fasteners 727, 729 and male Velcro fasteners 737 and 739, when the center portion 733 is passed under the crotch region and onto the top surface 723A of the back waist region, the male Velcro fasteners 737, 739 will be engaged onto the female Velcro fasteners 727 and 729 such that the male Velcro fastener 737 engages the female Velcro fastener 729 and the male Velcro fastener 739 engages the female Velcro fastener 727.

Also, as in the embodiment illustrated in the previous embodiments, the mid-region of the back waist may be a non-woven fabric having a female surface adapted to be engaged into the male fasteners 737, 739. Other variations and modifications readily suggest themselves from the description herein.

The materials and fabrics used in the making the absorbent article of the present invention are generally of the type and variety known in the art and are described, for example, in United States Patent Nos. 4,695,278 and 4,795,454 and in the aforementioned copending, commonly assigned application serial number 09/149,265, the disclosures of which are fully incorporated herein by reverence. Thus the liquid pervious cover sheet is a compliant soft material which is skin friendly and does not cause rash or irritation. Such materials include porous foams, reticulated foams, plastics, natural fibers such as wood or cotton fibers, synthetic fibers made of polyester or polypropylene available from First Quality Fibers, Inc., McElhattan, PA, or made from a combination of such materials.

The absorbent pad or core may be manufactured from a wide variety of liquid absorbent materials of the type usually used in manufacturing disposable diapers and other absorbent articles. Such materials include comminuted wood pulp, creped cellulose wadding, absorbent foams and sponges, super absorbent polymers, or a combination of said materials.

The acquisition layer is usually made of chemically bonded nonwoven polypropylene available from American Nonwovens, Columbus, Missouri. Preferably, the width of this layer is substantially the same as the width of the absorbent core. This core may be made of wood pulp fibers and super absorbent polymers such as IM 7000 series available from Clarian Products, Inc., Portsmouth, Inc., VA, and Chemdal 200 series, available from Chemdal, Inc. Palantine, Illinois. Alternatively, the absorbent core may be made of dual layer construction, in which case, the absorbent polymer may be securely positioned between each layer of the absorbent material.

The film backing is usually a polyethylene layer which is liquid, air and preferably vapor impermeable, and is placed under the absorbent core to prevent the body exudates from leaking and otherwise soiling the user's bed and clothing. The width and length of the backing film are generally at least equal to the width and length of the absorbent core. Polyethylenes suitable as backing film for the purpose of this invention are available from Clopay Plastics, Cincinnati, Ohio. The backsheet is also preferably made of spunbond nonwoven polypropylene and is usually coextensive with the backing film. In general, however, the various layers are of the type and materials well known in the diaper industry and within the scope and knowledge of those versed in the art.

## Claims

1. An integral disposable elasticized absorbent article comprising:
(a) an absorbent body having a front waist portion, a back waist portion, crotch portion and a pair of spaced apart leg openings;
(b) a fastening region intermediate said crotch portion and said front waist portion, and
(c) an elasticized band member attached to said back waist portion, said elasticized band member having a left hand band portion and a right hand band portion, each of said band portions extending angularly toward said fastening region, each of said band portions having an end portion adapted to be engaged to one another and to said fastening region.

2. An integral disposable elasticized absorbent article as in claim 1 additionally comprising:
(d) a liquid pervious top layer facing the body of the wearer,
(e) a liquid impervious back layer substantially coextensive with said liquid pervious layer,
(f) a liquid absorbent layer disposed between said liquid pervious top layer and liquid impervious back layer, said liquid absorbent layer being substantially coextensive with said liquid pervious top layer and said liquid impervious back layer and being sealed to said layers.

3. An integral disposable elasticized absorbent article as in claim 1 or 2 wherein each of said elasticized band portions comprises an end portion having an outer non-adherent surface and an inner adherent surface, and wherein said fastening region has an outer non-adherent surface removably fastenable to one of said inner surfaces of said band portions.

4. An integral disposable elasticized absorbent article as in any of the claims 1-3, wherein said elasticized band member comprises at least one elasticized band element formed into a loop extending from one end of the elasticized band member to the other end of the elasticized band member.

5. A disposable elasticized absorbent article comprising:
(a) an absorbent body having a front waist portion, a back waist portion, a crotch portion and a pair of spaced apart leg openings;
(b) a fastening region intermediate said crotch portion and said front waist portion, and
(c) a separate elasticized band member attached to said back waist portion said elasticized band member having a left hand band portion and a right hand band portion, each of said band portions extending angularly toward said fastening region, each of said band portions having an end portion adapted to be engaged to one another and to said fastening region.

6. A disposable elasticized absorbent article as in claim 5 further comprising:
(d) a liquid pervious top layer facing the body of the wearer;
(e) a liquid impervious back layer substantially coextensive with said liquid pervious layer;
(f) a liquid absorbent layer disposed between said liquid pervious top layer and liquid impervious back layer, said liquid absorbent layer being substantially coextensive with said liquid pervious top layer and said liquid impervious layer and being sealed to said layers.

7. A disposable elasticized absorbent article as in claim 5 or 6 wherein each of said elasticized band portions comprised an end portion having an outer non-adherent surface and an inner adherent surface, and wherein said fastening region has an outer non-adherent surface removably fastenable to one of said inner surfaces of said band portions.

8. A disposable elasticized absorbent article as in any of the claims 5-7 wherein said elasticized band member comprises at least one elasticized band element formed into a loop extending from one end of the elasticized band member to the other end of the elasticized band member.

9. A two-piece disposable elasticized absorbent article comprising, as a first separate piece, an absorbent body having a front waist portion, a back waist portion, a crotch portion, a pair of spaced apart leg openings, and a fastening region intermediate said crotch portion and said back waist portion,
in combination therewith, as a second separate piece, an elasticized band member attachable to said back waist portion, said elasticized band member having a left hand band portion and a right hand band portion, each of said band portions extending angularly toward said fastening region, each of said band portions having an end portion adapted to be engaged to one another and to said fastening region.

10. A two-piece disposable absorbent article comprising, as a separate first piece, an absorbent body having a front waist portion, a back waist portion, a crotch portion, a pair of spaced apart leg openings, a fastening region intermediate said crotch portion and said front waist portion of said absorbent article, a liquid pervious top layer facing the body of the wearer, a liquid impervious back layer substantially coextensive with said liquid pervious layer, a liquid absorbent layer disposed between said liquid pervious top layer and liquid impervious back layer, said liquid absorbent layer being substantially coextensive with said liquid pervious top layer and said liquid impervious back layer and being sealed to said layers,
in combination therewith, as a second separate piece, an elasticized band member attachable to said back waist portion, said elasticized band member having a left hand band portion and a right hand band portion, each of said band portions extending from the back waist portion angularly toward said fastening region, and each of said band portions having an end portion adapted to be engaged to each other and to said fastening region.

11. A two-piece absorbent article as in claim 9 or 10 wherein each of said elasticized band portions comprised an end portion having an outer non-adherent surface and an inner adherent surface, and wherein said fastening region has an outer non-adherent surface removably fastenable to one of said inner surfaces of said band portions.

12. A two-piece disposable absorbent article as in claim 10 or 11 wherein said elasticized band member comprises at least one elasticized band element formed into a loop extending from one end of the elasticized band member to the other end of the elasticized band member.

13. An integral disposable elasticized absorbent article comprising:
(a) an absorbent body having a front waist portion, a back waist portion, crotch portion and a pair of spaced apart leg openings, said absorbent article being adapted for wearing around the hip portion of the wearer,
(b) a fastening region intermediate said crotch portion and said front waist portion, and
(c) a left hand band member and a right hand band member, each of said band members extending angularly from its respective hip portion toward said fastening region, each of said band members having an end portion adapted to be engaged to one another and to said fastening region.

14. An integral disposable absorbent article comprising:
(a) an absorbent body having a front waist portion, a back waist portion, crotch portion and a pair of spaced apart leg openings, said absorbent body being adapted for wearing around the hip portion of the wearer;
(b) a fastening region intermediate said crotch portion and said front waist portion of said absorbent article;
(c) a liquid pervious top layer facing the body of the wearer;
(d) a liquid impervious back layer substantially coextensive with said liquid pervious layer;
(e) a liquid absorbent layer disposed between said liquid pervious top layer and liquid impervious back layer, said liquid absorbent layer being substantially coextensive with said liquid pervious top layer and said liquid impervious back layer and being sealed to said layers, and
(f) a left hand band member and a right hand band member, each of said band members extending angularly from its respective hip portion toward said fastening region, each of said band portions having an end portion adapted to be engaged to one another and to said fastening region.

15. An absorbent article having a chassis comprising two portions which together define a generally T-shaped configuration when said chassis is viewed in stretched position; a first portion having opposed lateral segments adapted to be wrapped around the waist of the wearer of said article and overlap on each other, a second portion having a distal end and a proximal end disposed generally vertically relative to said first portion and adapted to be passed under the crotch of the wearer and folded upwardly and over said overlapped portions:
one male fastening means on one of said lateral segments, and at least one female fastening means on said other lateral segment, such that when said lateral segments overlap each other said male fastening means engages said female fastening means;
a pair of opposed spaced apart male fastening means at the distal end of said second portion, and a pair of spaced apart opposed female fastening means at said first portion such that when said lateral segments are overlapped and said second portion is folded over said overlapped portion, each of said pair of male fastening means engage a correspondingly aligned one of said pair of female fastening means.

16. A generally T-shaped absorbent article as in claim 15 wherein at least one of said lateral segments has a female fastening surface adapted to be engaged into the male fastening means.

17. An absorbent article as in claim 15 wherein said second portion is shorter in length relative to said first portion.

18. An absorbent article as in claim 16 wherein said second portion is shorter in length relative to said first portion.

19. An absorbent article as in any of the claims 15-18 wherein said first portion is at least partly elasticated.

20. An absorbent article having a chassis comprising two portions which together define a generally T-shaped configuration when said chassis is viewed in stretched position; a first portion having a female surface, and a second portion disposed generally vertically relative to said first portion and adapted to be passed under the crotch of the wearer and folded upwardly and over said first portion;
said first portion having opposed lateral segments adapted to be wrapped around the torso of the wearer,
at least one male fastening means on one of said lateral segments,
a pair of opposed spaced apart male fastening means at the distal end of said second portion,
such that when said lateral segments are in overlapped position and said second portion is folded over said overlapped portions, said male fastening means engages said female surface of said first portion.

21. An absorbent article as in claim 20 wherein said second portion is shorter in length relative to said first portion.

22. An absorbent article as in claim 20 or 21 wherein said first portion is at least partly elasticated.

23. An absorbent article having a chassis comprising two portions which together define a generally L-shaped configuration when said chassis is viewed in stretched position; a first portion and a second portion disposed generally vertically relative to said first portion, said first portion having a laterally extending segment and said second portion having a distal end and a proximal end, and a flared out portion extending from said proximal end disposed in opposed spaced apart relation relative to said laterally extending segment such as to define said generally L-shaped configuration;
at least one male fastening means on said laterally extending segment;
at least three spaced apart female fastening means disposed in spaced apart relations on said first portion;
a pair of opposed and spaced apart male fastening means on said second portion at the distal end thereof,
such that when said laterally extending segment and said flared out portion is wrapped around the torso of the wearer, said male fastening means on said laterally extending segment engages the female fastening means on said flared out portion, and when said second portion is folded over onto said wrapped around portion, said male fastening means at the distal end of said first portion engage the correspondingly aligned female fastening means.

24. An integral disposable article as in claim 23 wherein said first portion has a female surface adapted to be engaged into said male fastening means when said second portion is folded over said female surface of said first portion.

25. An integral disposable article as in claim 23 wherein said first portion is at least partly elasticated.

26. An integral disposable article as in claim 24 wherein said first portion is at least partly elasticated.
